# EUROPEAN PATENT SPECIFICATION

(11) **EP 1 716 877 B1**
(45) Date of publication and mention of the grant of the patent: **16.06.2010**
(21) Application number: 06016813.5
(22) Date of filing: 18.12.1998
(51) Int. Cl.: A61M 5/00, A61B 17/00

(54) **Two component dispenser system**
Aus zwei Elementen bestehende Abgabevorrichtung
Système de distribution à deux éléments

(30) Priority: 19.12.1997 US 68220 P; 19.12.1997 US 68218 P
(43) Date of publication of application: 02.11.2006
(62) Divisional of application: 98966025.3
(73) Proprietor: United States Surgical Corporation, Norwalk, Connecticut 06856 (US); Roby, Mark S., Kilingworth, CT 06419 (US); Aranyi, Ernie, Easton, CT 06612 (US); Yogami, Richard, Ridgerield, CT 06877 (US); Tovey, Jonathan H., East Hampton, CT 06424 (US)
(72) Inventor: Roby, Mark, S., Kilingworth, CT 06419 (US); Aranyi, Ernie, Easton, CT 06612 (US); Yogami, Richard, Ridgerield, CT 06877 (US); Tovey, H.,Jonathan, East Hampton, CT 06424 (US)
(74) Representative: HOFFMANN EITLE

(56) References cited:
- WO-A-97/33646
- GB-A- 2 192 354
- US-A- 4 846 405
- US-A- 5 648 265
- US-A- 5 656 035

## Description

### BACKGROUND

### 1. Technical Field

The disclosure relates generally to an applicator for applying a tissue sealant based on human or animal proteins and more particularly to an apparatus for applying an adhesive formed by combining solutions of the proteins to tissues or organs for sealing wounds, stopping bleeding and the like.

### 2. Description of Related Art

A fibrin sealant is a biological adhesive formed by mixing two protein components, namely, fibrinogen and thrombin. Each protein component is derived from human plasma and is subjected to virus elimination procedures. The components are typically individually dehydrated and stored in separate vials as sterile freeze-dried powders.

It is known that purified fibrinogen and thrombin, together with a variety of known adjuvants, can be combined in vitro to produce a polymer having great potential benefit, both as a hemostatic agent and as a tissue adhesive. Because of the rapid polymerization upon intimate interaction of fibrinogen and thrombin, it is important to maintain these two blood proteins separate until applied at the application site. These protein solutions are generally delivered by devices such as a dual syringe apparatus. One dual syringe apparatus for applying a fibrinogen-based tissue adhesive is disclosed in U.S. Pat. No. 4,359,049 to Redl et al. Redl et al, disclose a mechanism in which two standardized one-way syringes are held in a support having a common actuating means. The dispensing end of each syringe is inserted into a collection manifold where the two components are mixed. The components are then dispensed through a common needle capable of covering a limited area of the application site.

However, it is sometimes desirable or necessary to cover a broad area of a wound, either to stop bleeding, to fix tissue or to prevent infection and sometimes it is desirable to cover a limited area. It is also desirable to prevent the two components from mixing within the dispensing device.

Further, all known devices for dispensing solutions of fibrinogen and thrombin require the addition of these proteins in powdered form to the body of the syringe. This makes the proteins susceptible to contamination by impurities which may enter the syringe body. Further still, the use of the syringe body to mix the proteins with water to create the protein solutions can cause the solutions to leak out from either the dispensing end of each syringe or the proximal end of the syringe body.

Additionally, a dual syringe apparatus for the application of fibrinogen and thrombin solutions to an application site generally contains several parts, such as a syringe plunger, a "Y" manifold connector, a dispensing needle, a syringe holder, syringe needles, and conduits for transporting the solutions to the dispensing needle. Therefore, known fibrin sealant applicators, such as disclosed in U.S. Patent to Redl et al. discussed above, and in U.S. Patent Nos. 4,874,368 to Miller et al. and 4,979,942 to Wolf et al. are difficult to reuse. The replenishment of the protein components typically require removing a clip which couples the syringe plunger, removing the syringe plunger, detaching the syringes from the "Y" connector, removing the syringes from the holder, inserting new syringes, affixing the syringes to the "Y" connector, adding fibrinogen to one syringe and thrombin to another syringe, adding sterile water to each syringe, replacing the syringe plunger, replacing the plunger clip, and mixing the solutions. In an application where time is of the essence, such a lengthy replenishing process is impractical and cumbersome.

US 5,648,265 discloses an apparatus for one-step preparation of a fibrinogen adhesive composition.

The two part form of appended claim 1 is based on this document.

GB 2192354 discloses a dispersing apparatus for a two part chemically reactive adhesive.

### SUMMARY

An applicator is provided for dispensing a first and a second component of a biological adhesive. The applicator includes a housing configured to receive a plurality of collapsible reservoirs and a plurality of vials each having a sealable opening therein; a first conduit assembly having a proximal end configured for respective fluid communication with one of the vials, an intermediate portion made up at least partially by one of the collapsible reservoirs and a distal end configured to be open to the environment; a second conduit assembly having a proximal end configured for respective fluid communication with one of the vials, an intermediate portion made up at least partially by one of the collapsible reservoirs and a distal end configured to be open to the environment; an activator assembly provided on the housing having an activator moveable from a first position to a second position to simultaneously compress each of the plurality of reservoirs to dispense the biological adhesive components through the first and second conduits from respective distal ends thereof; and a valve assembly having a first valve operatively associated with the proximal ends of the first and second conduit assemblies for opening and closing the conduits and a second valve independent of the first valve and operatively associated with the distal ends of the first and second conduit assemblies for opening and closing the conduits adjacent the distal ends.

The housing further includes an elongated body portion defining a longitudinal axis for enclosing a portion of the conduit assemblies therein. An applicator tip having two separate channels in communication with the conduits may be provided on a distal end of the elongated body portion for dispensing the components at the application site. The first and second components are preferably fibrinogen and thrombin which intermix to form a fibrin sealant.

### RRIEF DESCRIPTION OF THE DRAWINGS

Various embodiments are described herein with reference to the drawings, wherein:
FIG. 1 is a perspective view of an exemplary of a fibrin sealant applicator having a deflection assembly;
FIG. 1A is an enlarged perspective view of a distal end of the fibrin sealant applicator of FIG. 1 with a control lever of the deflection assembly in a retracted position;
FIG. 1B is an enlarged perspective view of a distal end of the fibrin sealant applicator of FIG. 1 with the control lever of the deflection assembly in an advanced position;
FIG. 2 is a perspective exploded view of the applicator of FIG. 1;
FIG. 2A is an enlarged view of a ratchet member on an activator assembly shown by FIG. 1;
FIG. 2B is an enlarged view of the distal end of the fibrin sealant applicator illustrating the assembly of the deflection assembly to the applicator of FIG. 1;
FIG. 2C is an enlarged view of the distal end of the fibrin sealant applicator illustrating the assembly of an alternative exemple of the deflection assembly;
FIG. 3 is a cross-sectional view of the fibrin sealant applicator with a deflection member of the deflection assembly in a retracted position;
FIG. 4 is a cross-sectional view of the distal end of the fibrin sealant applicator with the deflection member of the deflection assembly in an advanced position;
FIG. 5 is a perspective view of an alternative embodiment of a fibrin sealant applicator in accordance with the present invention;
FIG. 6A is a cross-sectional view taken along line 6A-6A in FIG. 5 with a first valve in an open position;
FIG. 6B is a cross-sectional view taken along line 6A-6A in FIG. 5 with the first valve in an closed position; and
FIGS. 7A and 7B are flow charts depicting the method of use of the fibrin sealant applicator of FIG. 5.

### DETAILED DESCRIPTION

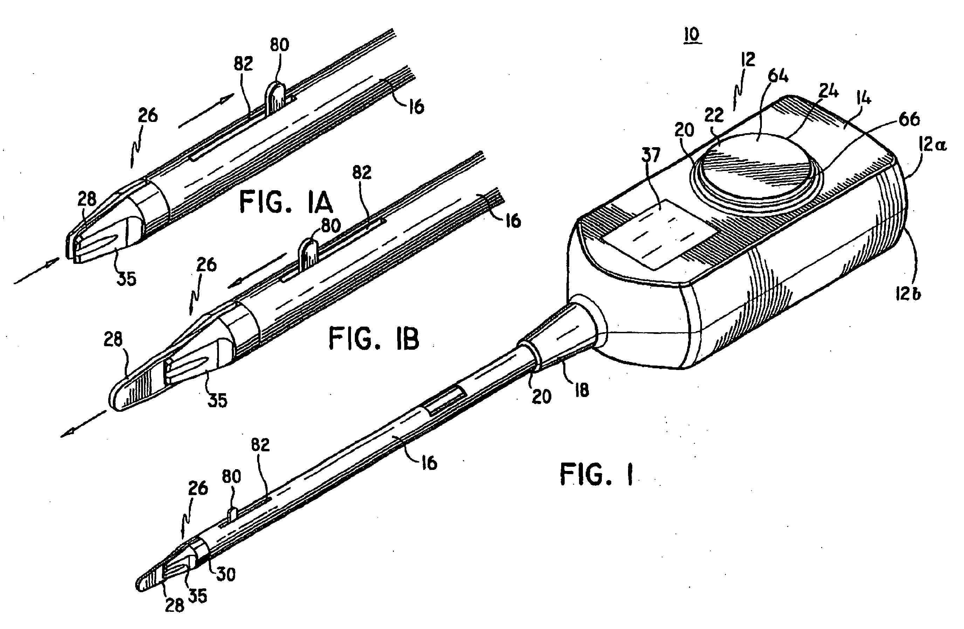

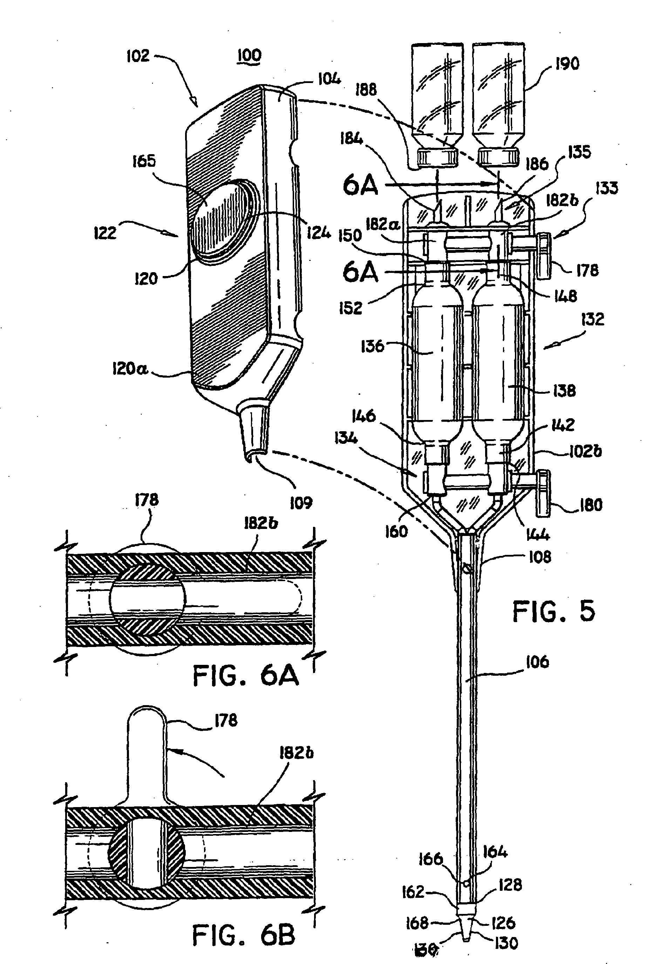

The applicator disclosed herein with reference to figures 1 to 4 is presented to give useful information to aid a complete understanding of the invention. The embodiment disclosed herein with reference to figures 5 to 7 is a preferred embodiment of the claimed invention.

Referring to FIG. 1, a fibrin sealant applicator is designated generally by numeral 10 and includes a housing 12 having a housing head 14 and an elongated body portion 16 defining a longitudinal axis. Housing head 14 contains a conically-shaped distal end 18 having a bore 20 in the center thereof dimensioned to receive body portion 16. While housing head 14 is shown as being rectangular, it is understood that other shapes that contribute to the ease of gripping and controlling the applicator 10 may be used.

Opening 20 is configured for receiving an activator assembly 22 having an activator 24 for effectuating the dispensing of biological components as further described below. A deflection assembly 26 is provided having a deflection member 28 at a distal end 30 of body portion 16. Deflection assembly 26 provides longitudinal movement of deflection member 28 to effectuate various dispensing methods of the biological components, such as spraying and dripping as discussed below. FIG. 1A shows deflection member 28 in the retracted position and FIG. 1B shows deflection member 28 in the advanced position. Deflection member 28 is preferably manufactured from a non-stick material such as PTFE.

In a preferred example, the biological components are a fibrinogen solution and a thrombin solution which intermix to form a fibrin sealant. It is to be understood, however, that other biological fluids may be substituted, depending upon the choice of mixture that is to be dispensed.

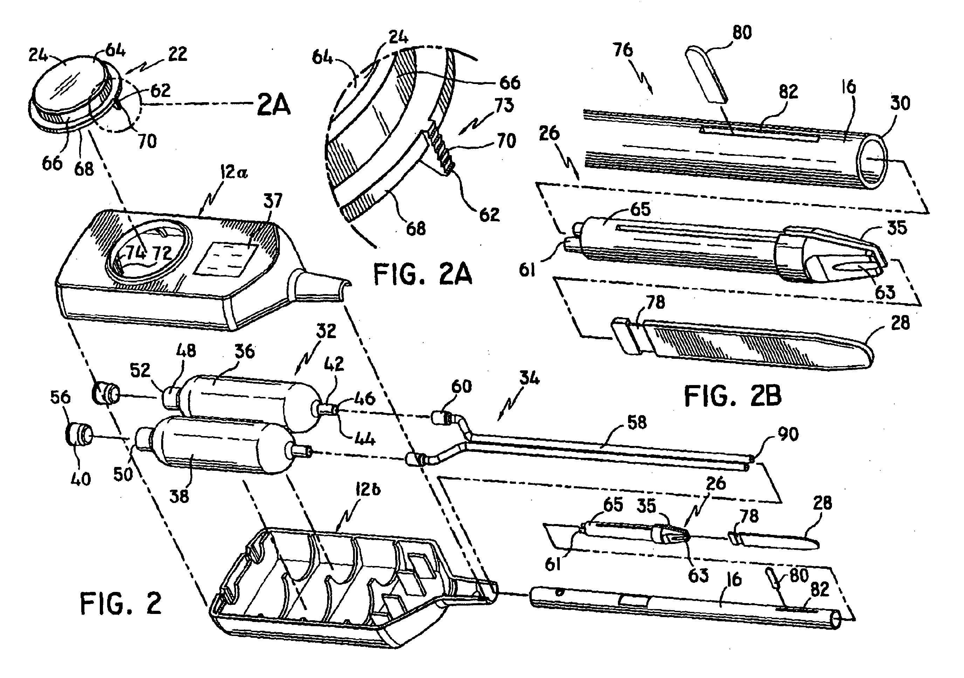

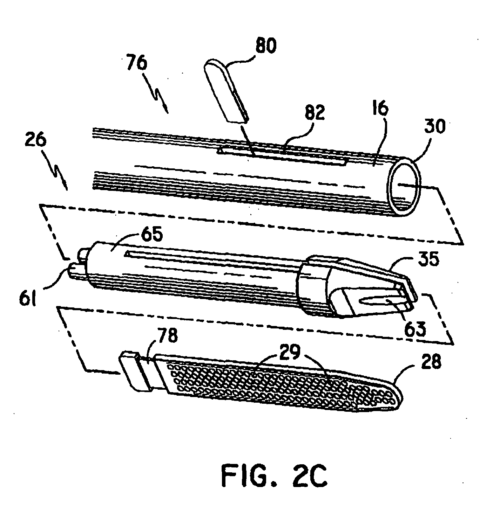

The internal components of housing 12 will now be discussed in detail with reference to FIGS. 2-2C. As shown in FIG. 2, housing 12 is formed from molded housing half sections 12a and 12b which are formed with internal partitions configured to properly align the internal components of applicator 10 with respect to each other and to prevent movement of the components. The internal components of applicator 10 include a reservoir assembly 32 and a conduit assembly 34. The two assemblies are interrelated with each other and with activator assembly 22 and deflection assembly 26 discussed above.

Reservoir assembly 32 includes first and second reservoirs 36, 38 and two plugs 40. First reservoir 36 and second reservoir 38 are preferably constructed from a flexible material and contain the first and second biological components, respectively. First and second reservoirs 36, 38 are identical for encasing an equal volumetric amount of their respective protein solution as compared to the other reservoir. It is contemplated to provide a different color for each reservoir to easily recognize the reservoir containing fibrinogen and the reservoir containing thrombin. It is further contemplated to provide a different shape for each reservoir for the same purpose. However, the volumetric amount stored within first reservoir 36 should be equal to the volumetric amount stored within second reservoir 38 to rnaintain a pre-determined fibrinogen to thrombin solution ratio, which is typically a 1:1 ratio.

It is still further contemplated that first and second reservoirs 36, 38 are manufactured from a transparent plastic for being able to view the amount of solution and to determine if the solution has been sufficiently intermixed before being dispensed on the application site. It is further contemplated to provide calibration markings on first and second reservoirs 36, 38. It is additionally contemplated that reservoir assembly 32 is permanently affixed to conduit assembly 34. Reservoir assembly 32 and conduit assembly 34 can be disposed of after each use and new reservoir and conduit assemblies can be fitted to applicator 10. A window 37 on housing half-section 12a will also permit a user to view the contents within first and second reservoirs 36, 38.

First and second reservoirs 36, 38 include a first cylindrical extension 42 having a central throughbore 44 at a distal end 46, a second cylindrical extension 48 having a central throughbore 50 at a proximal end 52. Central throughbore 50 is used for placing the biological components in reservoirs 36, 38. Plug 40 is used to vacuum seal central throughbore 50 to prevent contamination of the biological components. Plug 40 includes a silicon surface 56 capable of being penetrated by a syringe needle for adding a liquid, preferably sterile water, within reservoirs 36. 38 to intermix with the biological components to form protein solutions. The protein solutions are kept separated to prevent intermixing and the creation of a fibrin sealant within applicator 10. Upon exertion of pressure on activator 24, the components are forced through conduit assembly 34 to applicator tip 35. The addition of water within reservoirs 36, 38 and the dispensing of protein solutions on the application site are further discussed below.

It is to be understood that applicator 10 could be fitted with any of a number of different reservoirs, including, without limitation, syringes, bags or tubing. Furthermore, although the reservoir assembly 32 has but two reservoirs, it is to be understood that additional reservoirs containing other solutions can be incorporated within applicator 10.

Conduit assembly 34 includes two conduits 58 each having a nozzle 60 for matingly engaging cylindrical extension 42 on first and second reservoirs 36, 38 for connecting conduit assembly 34 to reservoir assembly 32. Conduit assembly 34 is mounted within housing 12. Two applicator nozzles 61 are press fitted to distal end surface 90 of conduits 58 for dispensing the protein solutions therefrom. Nozzles 61 lead to corresponding dispensing nozzles 63 on applicator tip 35 for dispensing the components in a spray-like manner. Applicator tip 35 is preferably manufactured from a non-stick material such as PTFE. A tubular extension 65 is provided to applicator tip 35 for press fitting to body portion 16.

FIG. 2A is an enlarged view of a portion of activator assembly 22. As described in greater detail below, activator assembly 22 controls the pressure exerted on reservoirs 36, 38, and includes activator 24 and a ratchet member 62. Activator 24 includes an activation area 64, a shaft 66, and a disc 68. Shaft 66 connects activation area 64 with disc 68. Ratchet member 62 extends downwardly from disc 68 and includes teeth 70 for engaging teeth 72 on an inner extension 74 of housing 12 to form structure for controlling the position of activator 24. The control structure is a ratchet mechanism 73. Ratchet member 62 is preferably formed integral with disc 68. Activator 24 may be formed with a transparent material or with a transparent window therein to permit viewing of the internal components of applicator 10.

Deflection assembly 26, as mentioned above includes deflection member 28, and a longitudinal advancement mechanism 76 having a control rod 78 operatively associated with a control lever 80 to distally advance and proximally retract deflection member 28 as control lever 80 is moved along slot 82. It is contemplated to provide a plurality of locking positions to deflection member 28 along control rod 78 for changing the length of deflection member 28 extending beyond applicator tip 35. FIG. 2B is an enlarged view of deflection member 28 showing its connection to control lever 80. FIG. 2C is an enlarged view of an alternative deflection member 28 having a plurality of holes 29 for intermixing the two protein solutions before they are dispensed on the application site.

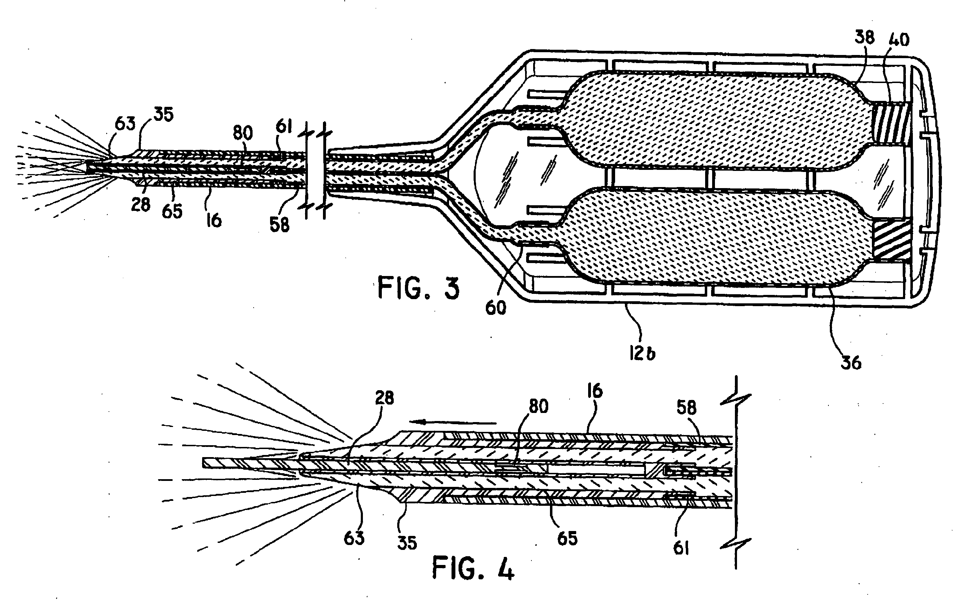

The operation of applicator 10 with particular attention to changing the amount of area covered by the dispensed adhesive will now be described in detail with reference to FIGS. 3 and 4. As described in U.S. Patent Application Serial No. 08/792,535, now published as US 2001/016709 A, the solutions are dispensed by exerting pressure to activation area 64. This causes ratchet mechanism 73 to guide activator 24 downwardly and to force shaft 66 further into housing 12. As shaft 66 enters housing 12, ratchet mechanism 73 and disc 68 compress reservoirs 36, 38 to dispense each protein solution via a corresponding nozzle 60 into conduit assembly 34. When ceasing to exert pressure to activation area 64, activator 24 is prevented from returning to the inactivated state by ratchet mechanism 73. As a result air cannot be sucked into reservoirs 36, 38 causing difficulty in further compressing reservoirs 36, 38.

While exerting pressure on activation area 64, control rod 78 can be moved distally and proximally to advance and retract deflection member 28, respectively. Deflection member 28 can also be held in a particular position throughout the dispensing procedure. When deflection member 28 is in the retracted position, as shown by FIG. 3, deflection of the spray is avoided. The solutions are therefore dispensed uniformly throughout the application site. On the other hand, when deflection member 28 is in the advanced position, as shown by FIG. 4, a portion of the spray is deflected. In this configuration, the solutions are preferably caused to drip from deflection member 28 onto the application site. Although the exemplary applicator has been described with a particular activator, conduit and deflection assemblies, it is understood that other similar assemblies may be employed to achieve the same functions.

For example, it is contemplated to provide different diameters for conduits 58 for dispensing the biological components in different ratios. Further, an activator assembly may be provided which uses pressurized gas to dispense the components from the reservoirs.

Referring to FIG. 5, a fibrin sealant applicator according to an embodiment of the present disclosure is shown. The presently disclosed applicator, designated generally by numeral 100, includes a housing 102 having a housing head 104 and an elongated body portion 106 defining a longitudinal axis. Housing head 104 contains a conically-shaped distal end 108 defining a bore 109 in the center thereof dimensioned to receive body portion 106. While housing head 104 is shown as being rectangular, it is understood that other shapes that contribute to the ease of gripping and controlling applicator 100 may be used.

Housing head 104 includes an opening 120 for receiving an activator assembly 122 having an activator 124 for effectuating the dispensing of biological components as further described below. An applicator tip 126 is provided at a distal end 128 of body portion 106 having two boresights 130 for dispensing biological components contained within housing head 104. As in the first embodiment, the biological components are a fibrinogen solution and a thrombin solution which intermix to form a fibrin sealant. It is to be understood, however, that other biological fluids may be substituted, depending upon the choice of mixture that is to be dispensed. Further, applicator tip 126 is preferably manufactured from a non-stick material such as PTFE.

The internal components of housing 102 will now be discussed in detail. As shown in FIG. 5, housing 102 is formed from molded housing half sections 102a and 102b which are formed with internal partitions configured to properly align the internal components of applicator 100 with respect to each other and to prevent movement of the components. The internal components of applicator 100 include a reservoir assembly 132, a valve assembly 133, a first conduit assembly 134, and a second conduit assembly 135. The four assemblies are interrelated with each other and with activator assembly 122 discussed above. For example, an intermediate portion of first and second conduit assemblies 134, 135 is made up at least partially by reservoir assembly 132.

Reservoir assembly 132 includes first and second reservoirs 136, 138. First reservoir 136 and second reservoir 138 are preferably constructed from a flexible material and contain the first and second biological components, respectively. It is contemplated to provide a window on housing half-section 102a to permit a user to view the contents within first and second reservoirs 136, 138 (see, e.g., FIG. 1).

First and second reservoirs 136, 138 include a first cylindrical extension 142 having a central throughbore 144 at a distal end 146, a second cylindrical extension 148 having a central throughbore 150 at a proximal end 152. Central throughbore 144 and central throughbore 150 of each respective reservoir are in alignment with corresponding first and second conduit assemblies 134, 135. Central throughbore 150 is used for placing the biological components in reservoirs 136, 138. Each conduit assembly includes a nozzle 160 at its intermediate portion for connecting to reservoirs 136, 138 via valve assembly 133.

A plug or sealable opening is preferably used to vacuum seal central throughbore 150 to prevent contamination of the biological components, similarly to reservoirs 36, 38. A piercer (not shown) within central throughbore 150 is used to pierce the plug when reservoirs 136, 138 are placed within housing 102. Alternatively, reservoirs 136, 138 having the protein components therein are welded to the proximal ends of first and second conduit assemblies 133,134 during manufacturing.

Applicator tip 126 includes a cylindrical proximal end 162 and an applicator head 168. Cylindrical proximal end 162 is press fitted to the distal end of elongated body portion 106 to provide communication between boresights 130 and conduit assemblies 134, 135. Each boresight 130 extends through applicator tip 126 to a respective conduit assembly for dispensing the protein solutions to the application site. Cylindrical proximal end 162 includes a clasping button 164 for matingly engaging a hole 166 in body portion 106 to secure applicator tip 126 to elongated body portion 106.

Activator assembly 122 controls the pressure exerted on reservoirs 136, 138, as further described below. Activator 124 may be formed with a transparent material or with a transparent window therein to permit viewing of the internal components of applicator 100.

First and second reservoirs 136, 138 of reservoir assembly 132 are preferably identical for encasing an equal volumetric amount of their respective protein solution. Further, it is contemplated, for example, to provide a different color for each reservoir to easily recognize the reservoir containing fibrinogen and the reservoir containing thrombin. Further still, it is contemplated to provide a different shape for each reservoir for the same purpose. However, the volumetric amount stored within first reservoir 136 should be equal to the volumetric amount stored within second reservoir 138 to maintain a pre-determined fibrinogen to thrombin solution ratio, which is typically a 1:1 ratio.

Valve assembly 133 includes first and second valves 178, 180. First valve 178 is operatively associated with the proximal ends of conduit assemblies 134, 135, which include pathways 182a, 182b. Second valve 180 is operatively associated with the intermediate portion of the conduit assemblies 134, 135. Pathways 182a, 182b lead to first and second reservoirs 136, 138 from first and second piercers 184, 186, respectively. First valve 178 opens and closes the two pathways 182a, 182b. As shown by FIG. 6A, when valve 178 is in the open position, pathway 182b is fully open to permit material to pass therethrough. When first valve 178 is in the closed position, as shown by FIG. 6B, pathway 182b is closed to prevent material to pass therethrough. The same operating mechanism is included for valve 180. The operating mechanism is a type of turnkey operation, which opens and closes first and second valves 178, 180 as it is turned clockwise and counter-clockwise, respectively.

Piercers 184, 186 are used to pierce a silicon surface 188 provided on vials 190. Vials 190 dispense sterile water within reservoirs 136, 138 for intermixing with the protein components therein to create protein solutions. The protein components may also be dispensed within reservoirs 136, 138 via vials similar to vials 190 or the like.

During the dispensing of sterile water within reservoirs 136, 138 to intermix with the protein components therein, first valve 178 is kept at an open position to keep pathways 182a, 182b open. Second valve 180 is kept at a closed position to prevent premature dispensing of the solutions via the distal ends of conduit assemblies 134, 135. When the protein solutions are ready to be dispensed, first valve 178 is at a closed position and second valve 180 is at an open position.

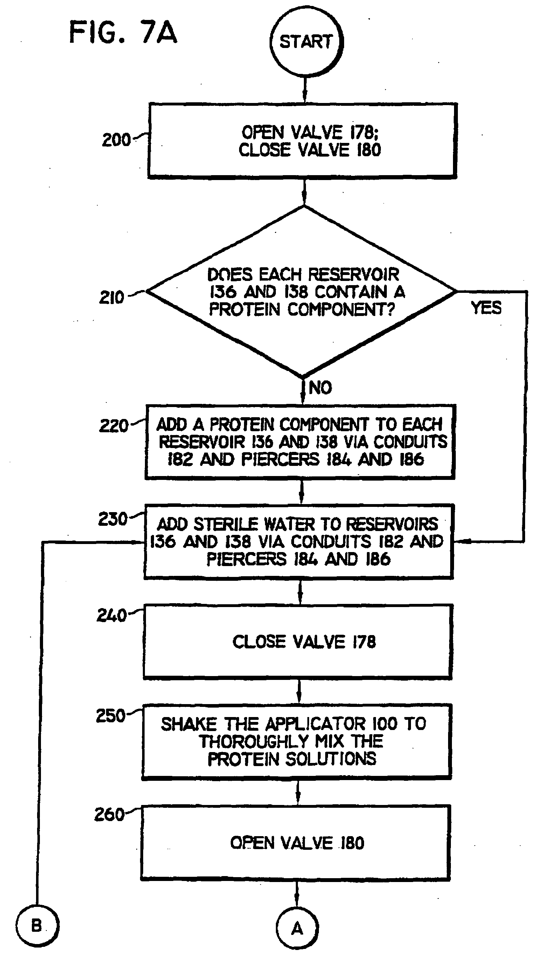

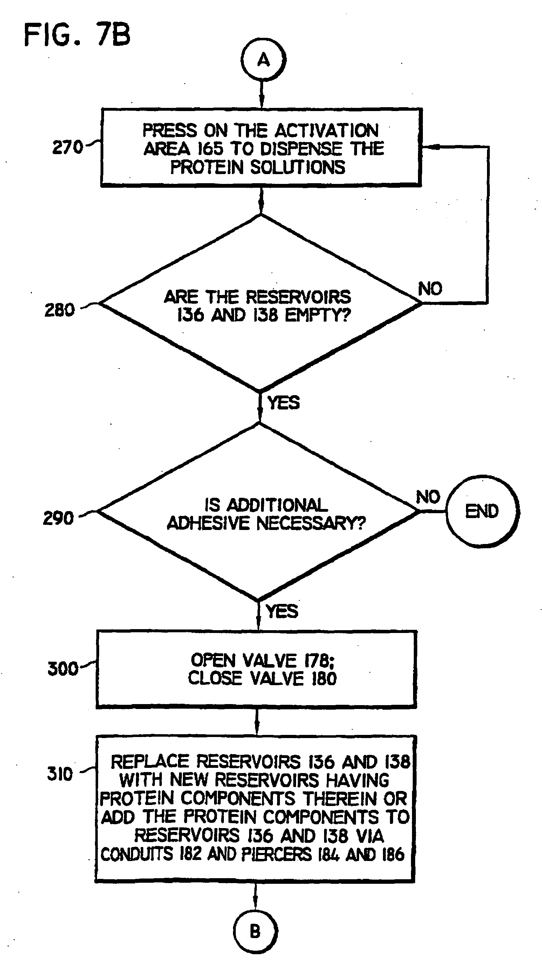

The above methodology will become more apparent with the following detailed description of the operation of applicator 100 with reference to FIGS. 7A and 7B. FIGS. 7A and 7B are flow chart diagrams depicting the operational steps of applicator 100. First, valve 178 is opened and valve 180 is closed (Block 200). If each reservoir 136, 138 contains a protein component (Block 210) the process continues with Block 230. Otherwise, a protein component is added to each reservoir 136, 138 via pathways 182a, 182b of conduit assemblies 134, 135 and pierces 184, 186 (Block 220). Sterile water is then added to reservoirs 136,138 (Block 230). FIrst valve 178 is then closed (Block 240). Applicator 100 is then shaken to thoroughly mix the components with the sterile water to form protein solutions (Block 250). Second valve 180 is then opened (Block 260).

Before dispensing the protein solutions, activator 124 is maintained in the inactivated state by a ratchet mechanism (similar to FIG. 2A) which has teeth for lockingly engaging teeth on an inner extension of housing 102. Activator 124 is activated by exerting pressure to activation area 165 (Block 270). This causes the ratchet mechanism to guide activator 124 downwardly into housing 102. As activator 124 enters housing 102, it compresses or pressurizes reservoirs 136, 138 to dispense the protein solutions via nozzles 160 located at the intermediate portion of conduit assemblies 135,135 towards elongated body portion 106.

When ceasing to exert pressure to activation area 165, activator 124 is prevented from returning to the inactivated state by the ratchet mechanism. As a result air cannot be sucked into reservoirs 136, 138 causing difficulty in further compressing reservoirs 136, 138, as described above with respect to the exemplary applicator of figure 2A. Further, the position of activator 124 with respect to housing half-section 102a provides a reference as to the amount of solution remaining in first and second reservoirs 136, 138. For example, when activator 124 is in a fully activated state, there is a small amount of solution left in first and second reservoirs 136, 138. In Block 280, the operator checks, e.g., via a window on having half-section 102a (see, e.g., FIG. 1), to determine if reservoirs 136, 138 are empty. If reservoirs 136, 138 are not empty, continued pressure on activation area 165 will dispense the remaining protein solutions. However, if reservoirs 136, 138 are empty, the operator can check to determine if additional adhesive is necessary (Block 290). If not, the process ends.

If additional adhesive is required, a replenishment process is initiated. First valve 178 is opened and second valve 180 is closed. (Block 300). Reservoirs 136, 138 can then be replaced or protein components may be added to reservoirs 136, 138 via pathways 182 and piercers 184. 186 (Block 310). The process then returns to Block 230 and is repeated.

Although the embodiments herein have been described with a particular activator assembly, it is understood that other similar assemblies may be employed. For example, an activator assembly may be provided which uses pressurized gas to dispense the components from the reservoirs. It is also contemplated to provide a different conduit assembly, for example, an assembly which has conduits with different diameters for allowing the biological components to be dispensed in different ratios.

Therefore, it is understood that various modifications may be made to the embodiments disclosed herein. For example, while specific preferred embodiments of the deflection, conduit, activator, ratchet and reservoir assemblies, have been described in detail, structures that perform substantially the same function in substantially the same way to achieve substantially the same result can also be used. Also, besides applying a fibrin sealant, the fibrin sealant applicator can be used to preform human or veterinary surgical procedures including applying antiseptics, medication and other similar procedures. Therefore, the above description should be construed as exemplifications of preferred embodiments without departing from the present invention as set forth in the appended claims.

## Claims

1. An applicator (100) for dispensing a first and second component of a biological adhesive, the applicator comprising:
a first conduit assembly (134) having a first conduit with a proximal end and a distal end;
a second conduit assembly (135) having a second conduit with a proximal end and a distal end;
a reservoir assembly (132) having a first reservoir (136) containing the first component and a second reservoir (138) containing the second component, the first reservoir being in communication with the first conduit and the second reservoir being in communication with the second conduit;
an activator assembly (122) having an activator (124) for imparting pressure to said first and second reservoirs to effect dispensing of said first and second components to said first and second conduits; and **characterised by**:
a valve assembly (133) having a first valve (178) for opening and closing the proximal ends of said conduits, to permit and prevent the introduction of said components in the recervoirs (136, 138), second valve (180) for opening and closing the distal ends of said conduits, to permit and prevent the dispensing of said components.

2. The applicator of Claim 1, wherein the first conduit assembly (134), the second conduit assembly (135), the reservoir assembly (132) and the valve assembly (133) are supported within a single housing (102).

3. The applicator of Claim 1, comprising
a housing (102) configured to receive a plurality of reservoirs (136, 138).

4. The applicator of any one of the preceding claims, wherein the first and second conduit assemblies (134, 135), the activator assembly (122) and the valve control assembly (133) are supported within a single housing (102).

5. The applicator of any one of Claims 1 to 4, comprising first and second vials (188, 190) each having a sealable opening therein, the first vial (188) in fluid communication with the proximal end of the first conduit and the second vial (190) in fluid communication with the proximal end of the second conduit.

6. The applicator of Claim 5, wherein the first and second conduit assemblies (134, 135), the reservoir assembly (132), the valve assembly (133) and the first and second vials (188, 190) are supported within a single housing (102).

7. The applicator of Claim 6, wherein the second valve (180) opens and closes said proximal ends of said pair of conduits to permit and prevent the introduction of components within said vials (188, 190) to said reservoirs (136, 138).

8. The applicator of any one of the preceding claims, wherein the first and second reservoirs (136, 138) are collapsible and the activator (124) is moveable from a first position to a second position to simultaneously compress each of said first and second reservoirs (136, 138) to dispense said biological adhesive components through said first and second conduits to respective distal ends thereof.

9. The applicator of Claim 8, wherein said activator assembly (122) includes control structure for restricting said activator (124) from returning to said first position after the activator (124) is moved from said first position.

10. The applicator of any one of the preceding claims, further comprising a pair of applicator nozzles (160) each being in fluid communication with said distal end of one of said conduits.

11. The applicator of any one of the preceding claims, wherein each of said conduits include independent distal exits.

12. The applicator of any one of the preceding claims, wherein said first component is a thrombin solution and said second component is a fibrinogen solution.

13. The applicator of Claim 12, wherein said biological adhesive comprises a predetermined ratio of said thrombin solution to said fibrinogen solution.

14. The applicator of any one of the preceding claims, wherein said housing includes a housing head (104) for storing said reservoirs (136, 138) therein and an elongated body portion (106) extending from said housing head (104).

15. The applicator of any one of the preceding claims, wherein the first conduit assembly (134) has a first intermediate portion made up at least partially by the first reservoir (136), and the second conduit assembly (135) has a second intermediate portion made up at least partially by the second reservoir (138).

## Patentansprüche

1. Applikator (100) zum Abgeben einer ersten und zweiten Komponente eines biologischen Klebstoffs, wobei der Applikator aufweist:
eine erste Leitungsanordnung (134) mit einer ersten Leitung mit einem proximalen Ende und einem distalen Ende;
eine zweite Leitungsanordnung (135) mit einer zweiten Leitung mit einem proximalen Ende und einem distalen Ende;
eine Reservoiranordnung (132) mit einem ersten Reservoir (136), das die erste Komponente enthält, und einem zweiten Reservoir (138), das die zweite Komponente enthält, wobei das erste Reservoir in Verbindung mit der ersten Leitung und das zweite Reservoir in Verbindung mit der zweiten Leitung ist;
eine Aktivatoranordnung (122) mit einem Aktivator (124), um das erste und zweite Reservoir einem Druck auszusetzen, um ein Abgeben der ersten und zweiten Komponente in die erste und zweite Leitung zu bewirken; und **gekennzeichnet durch**
eine Ventilanordnung (133) mit einem ersten Ventil (178) zum Öffnen und Schließen der proximalen Enden der Leitungen, um das Einführen der Komponenten in die Reservoirs (136, 138) zu erlauben und zu verhindern, und einem zweiten Ventil (180) zum Öffnen und Schließen der distalen Enden der Leitungen, um das Abgeben der Komponenten zu erlauben und zu verhindern.

2. Applikator nach Anspruch 1, wobei die erste Leitungsanordnung (134), die zweite Leitungsanordnung (135), die Reservoiranordnung (132) und die Ventilanordnung (133) innerhalb eines einzigen Gehäuses (102) gehalten sind.

3. Applikator nach Anspruch 1, umfassend ein Gehäuse (102), das zum Aufnehmen einer Mehrzahl von Reservoirs (136, 138) ausgestaltet ist.

4. Applikator nach einem der vorhergehenden Ansprüche, wobei die erste und zweite Leitungsanordnung (134, 135), die Aktivatoranordnung (122) und die Ventilsteuerungsanordnung (133) innerhalb eines einzigen Gehäuses (102) gehalten sind.

5. Applikator nach einem der Ansprüche 1 bis 4, aufweisend eine erste und zweite Ampulle (188, 190), die jeweils eine abdichtbare Öffnung darin haben, wobei die erste Ampulle (188) in Fluidverbindung mit dem proximalen Ende der ersten Leitung und die zweite Ampulle (190) in Fluidverbindung mit dem proximalen Ende der zweiten Leitung ist.

6. Applikator nach Anspruch 5, wobei die erste und zweite Leitungsanordnung (134, 135), die Reservoiranordnung (132), die Ventilanordnung (133) und die erste und zweite Ampulle (188, 190) innerhalb eines einzigen Gehäuses (102) gehalten sind.

7. Applikator nach Anspruch 6, wobei das zweite Ventil (180) die proximalen Enden des Paars von Leitungen öffnet und schließt, um das Einführen von Komponenten innerhalb der Ampullen (188, 190) in die Reservoirs (136, 138) zu erlauben und zu verhindern.

8. Applikator nach einem der vorhergehenden Ansprüche, wobei das erste und zweite Reservoir (136, 138) zusammenfaltbar sind und der Aktivator (124) aus einer ersten Position in eine zweite Position beweglich ist, um gleichzeitig jedes des ersten und zweiten Reservoirs (136, 138) zu komprimieren, um die biologischen Klebstoffkomponenten durch die erste und zweite Leitung zu den jeweiligen distalen Enden davon abzugeben.

9. Applikator nach Anspruch 8, wobei die Aktivatoranordnung (122) eine Steuerungsstruktur zum Beschränken des Aktivators (124) im Zurückkehren in die erste Position, nachdem der Aktivator (124) aus der ersten Position bewegt ist, enthält.

10. Applikator nach einem der vorhergehenden Ansprüche, ferner aufweisend ein Paar von Applikatordüsen (160), die jeweils in Fluidverbindung mit dem distalen Ende von einer der Leitungen sind.

11. Applikator nach einem der vorhergehenden Ansprüche, wobei jede der Leitungen unabhängige distale Ausgänge enthält.

12. Applikator nach einem der vorhergehenden Ansprüche, wobei die erste Komponente eine Thrombinlösung und die zweite Komponente eine Fibrinogenlösung ist.

13. Applikator nach Anspruch 12, wobei der biologische Klebstoff ein vorbestimmtes Verhältnis der Thrombinlösung zu der Fibrinogenlösung umfasst.

14. Applikator nach einem der vorhergehenden Ansprüche, wobei das Gehäuse einen Gehäusekopf (104) zum darin Lagern der Reservoirs (136, 138) und einen länglichen Körperabschnitt (106), der sich von dem Gehäusekopf (104) erstreckt, enthält.

15. Applikator nach einem der vorhergehenden Ansprüche, wobei die erste Leitungsanordnung (134) einen ersten Zwischenabschnitt, der zumindest teilweise durch das erste Reservoir (136) gebildet wird, aufweist und die zweite Leitungsanordnung (135) einen zweiten Zwischenabschnitt, der zumindest teilweise durch das zweite Reservoir (138) gebildet wird, aufweist.

## Revendications

1. Applicateur (100) pour distribuer un premier et deuxième composant d'un adhésif biologique, l'applicateur comprenant:
un premier ensemble de conduit (134) ayant un premier conduit avec une extrémité proximale et une extrémité distale;
un deuxième ensemble de conduit (136) ayant un deuxième conduit avec une extrémité proximale et une extrémité distale;
un ensemble de réservoirs (132) comportant un premier réservoir (136) contenant le premier composant et un deuxième réservoir (138) contenant le deuxième composant, le premier réservoir étant en communication avec le premier conduit, et le deuxième réservoir étant en communication avec le deuxième conduit;
un ensemble d'activateur (122) comportant un activateur (124) pour impartir une pression auxdits premier et deuxième réservoirs pour effectuer la distribution desdits premier et deuxième composants auxdits premier et deuxième conduits; et **caractérisé par**:
un ensemble de vannes (133) comportant une première vanne (178) pour ouvrir et fermer les extrémités proximales desdits conduits, pour permettre et empêcher l'introduction desdits composants dans les réservoirs (136, 138), et une deuxième vanne (180) pour ouvrir et fermer les extrémités distales desdits conduits, pour permettre et empêcher la distribution desdits composants.

2. Applicateur selon la revendication 1, dans lequel le premier ensemble de conduit (134), le deuxième ensemble de conduit (135), l'ensemble de réservoirs (132) et l'ensemble de vannes (133) sont supportés dans un seul boîtier (102).

3. Applicateur selon la revendication 1, comprenant
un boîtier (102) configuré pour recevoir une pluralité de réservoirs (136, 138).

4. Applicateur selon l'une quelconque des revendications précédentes, dans lequel les premier et deuxième ensembles de conduit (134, 135), l'ensemble d'activateur (122) et l'ensemble de commande de vannes (133) sont supportés dans un seul boîtier (102).

5. Applicateur selon l'une quelconque des revendications 1 à 4, comprenant des premier et deuxième flacons (188, 190), chacun ayant une ouverture pouvant être scellée dans celui-ci, le premier flacon (188) étant en communication fluidique avec l'extrémité proximale du premier conduit, et le deuxième flacon (190) en communication fluidique avec l'extrémité proximale du deuxième conduit.

6. Applicateur selon la revendication 5, dans lequel les premier et deuxième ensembles de conduit (134, 135), l'ensemble de réservoirs (132), l'ensemble de vannes (133) et les premier et deuxième flacons (188, 190) sont supportés dans un seul boîtier (102).

7. Applicateur selon la revendication 6, dans lequel la deuxième vanne (180) ouvre et ferme lesdites extrémités proximales de ladite paire de conduits pour permettre et empêcher une introduction de composants dans lesdits flacons (188, 190) auxdits réservoirs (136, 138).

8. Applicateur selon l'une quelconque des revendications précédentes, dans lequel les premier et deuxième réservoirs (136, 138) sont compressibles, et l'activateur (124) est déplaçable d'une première position à une deuxième position pour compresser simultanément chacun desdits premier et deuxième réservoirs (136, 138) pour distribuer lesdits composants d'adhésif biologique à travers lesdits premier et deuxième conduits aux extrémités distales respectives de ceux-ci.

9. Applicateur selon la revendication 8, dans lequel ledit ensemble d'activateur (122) comporte une structure de commande pour empêcher ledit activateur (124) de revenir à ladite première position après que l'activateur (124) a été déplacé de ladite première position.

10. Applicateur selon l'une quelconque des revendications précédentes, comprenant en outre une paire de buses d'applicateur (160) chacune étant en communication fluidique avec ladite extrémité distale d'un desdits conduits.

11. Applicateur selon l'une quelconque des revendications précédentes, dans lequel chacun desdits conduits comporte des sorties distales indépendantes.

12. Applicateur selon l'une quelconque des revendications précédentes, dans lequel ledit premier composant est une solution de thrombine, et ledit second composant est une solution de fibrinogène.

13. Applicateur selon la revendication 12, dans lequel ledit adhésif biologique comprend un rapport prédéterminé de ladite solution de thrombine à ladite solution de fibrinogène.

14. Applicateur selon l'une quelconque des revendications précédentes, dans lequel ledit boîtier comporte une tête de boîtier (104) pour stocker lesdits réservoirs (136, 138) dans celle-ci, et une portion de corps oblongue (106) s'étendant depuis ladite tête de boîtier (104).

15. Applicateur selon l'une quelconque des revendications précédentes, dans lequel le premier ensemble de conduit (134) possède une première portion intermédiaire constituée au moins partiellement par le premier réservoir (136), et le deuxième ensemble de conduit (135) possède une deuxième portion intermédiaire constituée au moins partiellement par le deuxième réservoir (138).
